# EUROPEAN PATENT APPLICATION

(11) **EP 4 059 488 A1**
(43) Date of publication of application: **21.09.2022**
(21) Application number: 21305340.8
(22) Date of filing: 19.03.2021
(51) Int. Cl.: A61K 8/06, A61K 8/55, A61K 8/81, A61K 8/92, A61Q 19/00

(54) **CONCENTRATED COMPOSITION AND USES OF SAME**

(71) Applicant: Johnson & Johnson Consumer Inc., Skillman, NJ 08558 (US)
(72) Inventor: HARDY, Carine, 27100 VAL DE REUIL (FR); PONTABRY, Alexandre, 27100 VAL DE REUIL (FR); SACLIER, Sebastien, 27100 VAL DE REUIL (FR); MOISSON, Stephanie, 1052 LE MONT SUR LAUSANNE (CH); SEKKAI, Sandhra, 93190 LIVRY GARGAN (FR)
(74) Representative: Carpmaels & Ransford LLP

(57) **Abstract**

Concentrated compositions, methods of using concentrated compositions, kits including concentrated compositions, and diluted forms of the concentrated compositions, which can be used to provide a benefit to the skin of the user.

## Description

### Field

The present invention relates to compositions that are in concentrated form as an emulsion, which are capable of forming useful skin care compositions after addition of an aqueous component with minimal effort and without the need for automated equipment or machinery.

### Background

Consumers desire skin care compositions for a variety of purposes, including, for example, to provide moisturization and/or cleansing of the skin. Skin care compositions must be capable of being applied to the skin of the user, and often require the presence of an aqueous component, such as water, to allow the user to apply the product to the skin easily and effectively. However, it is also desired to minimize packaging and/or shipping expenses, which can be increased when final compositions are packaged and shipped. It is therefore desirable to package and ship a concentrated product, which has a low water content as defined herein.

Currently, concentrated products are available commercially, and when they are to be used by consumers, the consumer adds a desired amount of water to the concentrated product to dilute it and render it suitable for use. However, some current concentrated products require a significant amount of water to prepare a final end product, and the resulting final product has a very low viscosity. While low viscosity products are suitable in some instances, they are not preferred for other skin care products, such as creams, balms, and other thicker products. Further, current products are not in the form of emulsions. Emulsions are traditionally difficult to use in concentrated form in a simple and easy mixing process. Emulsions typically require more vigorous mixing than that performed by a user's hand, requiring electronic mixing apparatuses to achieve mixing to provide a suitable end product.

There is a need for a skin care products which is prepared, packaged, and supplied to consumers in the form of a concentrated emulsion, where the concentrated emulsion may be diluted with a desired amount of water and mixed with simple methods by the consumer to form the final applicable skin care product, where the final skin care product has a suitably high viscosity.

### Summary

The present invention may include compositions, methods of making or using the compositions, and kits for providing and/or preparing the compositions described herein. Compositions described herein include concentrated compositions and diluted compositions, which are formed by combination of the concentrated composition and water or other aqueous material.

The invention may include a concentrated composition including from about 30% to about 60% by weight of water; at least two emulsifying agents: a first emulsifying agent and a second emulsifying agent, where the weight percent of each of the at least two emulsifying agents is from about 0.05% to about 7%; and from 0.1% to about 5% by weight of a thickener, and where the concentrated composition is an emulsion, and the emulsion is stable at room temperature.

The present invention may further include a diluted form of the concentrated composition, where the diluted form is capable of being applied to the skin and providing a benefit to the skin, such as moisturization of the skin. Dilution may be achieved through the addition of water and mixing, and the mixing may be achieved without the use of a motorized or electromechanical apparatus. The diluted form may be achieved through the mixing of water or other aqueous component. The diluted skin care product has a sufficiently high viscosity to be used as described herein, even after the addition of the aqueous component.

The present invention may additionally include a skin care product kit including a container including at least a first composition and a second composition, where the first composition includes a concentrated composition described herein, and the second composition includes at least one of a texture ingredient or a skin active ingredient. The first composition and the second composition in the kit can be mixed together with water, to obtain a skin care product capable of being applied to the skin of the user.

The invention may further include a method of forming a diluted skin care product, including the steps of combining together: a concentrated composition including: from about 30% to about 60% by weight of water, at least two emulsifying agents: a first emulsifying agent and a second emulsifying agent, where the weight percent of each of the at least two emulsifying agents is from about 0.05% to about 7%, and from 0.1% to about 5% by weight of a thickener; where the concentrated composition is an emulsion, where the emulsion is stable at room temperature; and an aqueous component. The combined concentrated composition and aqueous component are mixed with each other to form a diluted skin care product.

### Detailed Description

As used herein, a skin care product or skin care composition is a product that can be applied to the skin or hair of a user, providing a benefit to that user. The benefit can be, for example, hydration of the skin or hair or delivery of one or more therapeutic or benefit agents to the skin or hair, as described in more detail below. The skin care product can be, for example, a moisturizer or other skin beneficial product.

All percentages of ingredients or components described herein are by weight of the composition in which the ingredient is included.

The present invention relates to a skin care product, which is a composition useful for providing a benefit to the skin. The skin care product described herein is in two distinct forms, with each being described below. The intent of this innovation is to provide the skin care product in its first form, which can then be modified by the user to result in the skin care product in its second form. It is understood that a difference between the first form and the second form includes the addition of an aqueous material and mixing. Other components may be added if desired, as described below. Aqueous material, for example, may be water. As used herein, the term "water" may include distilled or filtered water, or it may include an aqueous liquid including water as its primary component, such as at least 95% water, or at least 98% water, or at least 99% water.

The first form of the skin care product is a concentrated product, which is considered to be the initial form and may be packaged and provided to a consumer. This is referred to as the concentrated skin care product or the concentrated composition. The first form is referred to herein interchangeably as the "first form" or the "concentrated form" of the skin care product. As a concentrated composition, the first form enjoys the benefit of having a smaller volume than the final form, thereby being capable of being shipped and sold in smaller packaging. As a concentrated composition, it includes a lower amount of aqueous material, such as water, than a non-concentrated product, which provides a number of benefits, such as reducing storage and shipping volume.

The second form of the skin care product is the final form of the skin care product, which is considered the skin-applied product, and which is achieved by diluting the first form of the skin care product with an aqueous component, as described herein. This second form is the form of the skin care product that is actually applied by the user on the skin or hair to achieve the benefit(s) of the skin care product. The second form of the skin care product includes the concentrated form of the skin care product and added aqueous material to achieve a suitable end product for use on the skin or hair. It is desired that the change from the first form of the skin care product to the second form of the skin care product is the result of the addition of a desired amount of aqueous material and the mixing of the first form and aqueous material, described below. In some aspects, there may be added one or more active ingredients to the concentrated composition and/or the diluted skin care product.

Thus, it may be that the second form of the skin care product consists of or consists essentially of the concentrated form of the skin care product and a desired amount of aqueous material. In an alternate aspect, the second form of the skin care product may consist of or consist essentially of the concentrated form of the skin care product, a desired amount of aqueous material, and one or more active ingredients. The difference between these aspects depends upon whether one or more active ingredients are maintained separately from the concentrated composition.

It may be desired that the second form of the skin care product has a volume that is larger than the concentrated form of the skin care product. In other words, the first form (the concentrated form) of the skin care product has a first volume, the second form (the diluted form) has a second volume, and the second volume is greater than the first volume.

The concentrated form of the skin care product is in the form of an emulsion, including skin care ingredients and an aqueous material, such as water. As used herein, the term "emulsion" means a mixture of two phases, including a first phase and second phase, where the first phase is dispersed into the second phase. In the concentrated form, one of the phases is in the form of a liquid, and is desirably an aqueous material, such as water. The second phase may be a liquid, a solid, or a flowable solid, and includes one or more skin care components described below, including, for example, emulsifying agents, thickeners, and humectants. The emulsion may be an oil-in-water emulsion. Concentrated emulsions are sought because it allows for a suitable mixture of components to be provided to consumers at a desirable viscosity at room temperature. This viscosity of the concentrated emulsion allows efficient mixing with water (dilution) by a consumer without the need for sophisticated or electronic mixing equipment as described below.

Put another way, the consumer may add water to dilute the concentrated product and thoroughly mix by hand, such as with a spoon, stick, or stirring rod, or even the user's fingers or hand. As used herein, the term "room temperature" refers to a temperature of approximately 20-25 degrees Celsius, or about 23 degrees Celsius.

The purpose of the present invention is to provide a concentrated composition which, when diluted with aqueous material, provides a resulting diluted product, where the diluted product still maintains stability and maintains a sufficiently high viscosity level to be used as described herein.

Concentrated products are available, however, typically concentrated products are not in the form of emulsions. In addition, typical concentrated products are diluted by a user to form a thin, low viscous end product. Typically, emulsions are not stable when they are diluted with water, particularly a higher weight amount of water than the weight amount of the starting emulsion. In fact, in some aspects of the present invention, there may be three times the amount of added water as compared to the amount of starting concentrated product, where the resulting diluted product is stable. In order to attempt to stabilize such previous emulsions, significant mixing force would be required, including a force far above hand-mixing. Addition of water to a typical emulsion will tend to break the emulsion, thereby changing the composition of the diluted composition to render it no longer useful for its purpose. For example, dilution of typical concentrated product, such as a cleanser, will decrease the viscosity of the skin care product to a level that is too low, which would make it difficult to use as a "thicker" product, such as a gel, balm or cream, or similar product where a higher viscosity is sought. Typical cleansing concentrates are therefore not capable of being diluted to form a gel, balm, or cream. Desirably, in order to be considered stable, a product includes one or more of the following attributes: a formulation that is visually unseparated and appears homogeneous, with no visible separation after one month or after three months; a formulation that has no noticeable odor change for after one month or after three months; and a formulation that has no pH shift (more than 0.5 change) as measured with any pH meter for after one month or after three months.

Thus, typical concentrated cleansers are different than the present invention, since the present invention is in the form of an emulsion and after dilution results in a thick, viscous product. The present invention is a concentrated emulsion, which is stable at room temperature and which is capable of being diluted by a consumer without breaking the emulsion, and which is diluted to form a product that has a resulting viscosity level above a desired level. It is understood that the resulting viscosity of the present invention results in a suitably high viscosity level, while prior attempts result in diluted products that have low viscosity levels. This allows the inventive product to be diluted and still used as a gel, balm, or cream.

Water is present in the concentrated composition in an amount lower than typically required in skin-applied products. For skin-applied non-concentrated products, such as creams, water is typically present in an amount greater than 70% by weight of the skin care product, and in some aspects may be present in an amount up to 90% by weight of the skin care product. The concentrated form of the present inventive skin care product includes water in an amount of from about 30% to about 60% by weight of the skin care product, or from about 35% to about 55% by weight of the skin care product, or from about 40% to about 50% by weight of the skin care product. The concentrated form of the skin care product may include an amount of water within the aforementioned ranges, including, by way of example, 37.08% or 56.8%.

Water is required in the concentrated form of the skin care product to form a water-based emulsion, and therefore sufficient water to form this emulsion is required. The water in the concentrated composition (to form the emulsion) should be sufficiently high to provide desired fluidity, but not so high to render it unstable or to render it no longer a concentrated product. Further, when too low an amount of water is included, the concentrated form of the skin care product is not acceptable since the resulting product is not fluid enough to allow for dilution and mixing by the user. For example, when water is included in an amount below about 30% by weight, the resulting product may include defects such as lumps or other visual defects. When water is included in too high an amount, the purpose of a concentrated product is not met, since the final product would not require dilution by the user. Therefore, it is desired that the concentrated form of the skin care product have water sufficient to form a stable emulsion, but not so much water so as to avoid the intent of the innovation.

It is preferred that the concentrated form of the skin care product have a viscosity that is sufficiently thick and viscous. While varying viscosity ranges may be acceptable, desired dynamic viscosity of the concentrated composition is from about 1,600 cP to about 13,000 cP at room temperature, or from about 1,600 cP to about 4,000 cP at room temperature, or about 1,600 cP to about 3,000 cP at room temperature, which may be measured by Physica Rheometer, at 20°C. The dynamic viscosity test is measured by using an Anton Paar MCR 301 rheometer, which is set up at 20 degrees C during 120 s, no shear rate, 3 measured every 10 seconds at 5 s⁻1, 9 measures every 4 seconds during increase from 5 s⁻¹ to 45 s⁻¹, 2 measures every 5 seconds at 45 s⁻¹, and the value to be recorded for viscosity is the second measure at 45 s⁻¹. The concentrated form of the skin care product may be diluted by the addition of water up to about 450%, while still providing a suitably high resulting viscosity as described herein. The dilution may result from the addition of water up to 50%, 75%, 100%, 200%, 300%, 400% or 450%, as described herein.

After dilution by the consumer as described below, the second form of the skin care product should have a resulting dynamic viscosity after mixing of above 300 cP at room temperature, or above 500 cP at room temperature, or above 750 cP as measured by Physica Rheometer, at 20°C. The resulting viscosity of the diluted product (the second form of the skin care product) may be up to about half of the viscosity of the concentrated form of the skin care product prior to dilution. Through the addition of a significant amount of water to the concentrated form, as described below, it would have been expected that the resulting diluted viscosity level would be lower, but the present inventors have discovered that the skin care compositions described herein maintain a sufficiently high dynamic viscosity (e.g., above 300 cP, above 500 cP, above 750 cP as measured by Physica Rheometer, at 20°C) even after addition of the desired amount of water and mixing.

The concentrated composition may have a pH that is approximately 5 to 7, or from 5.5 to 6.5.

The first form of the skin care product, being a concentrated form of the skin care product, provides benefits in that the volume of the concentrated composition is reduced compared to a product in a form that is intended to be applied to the skin. Thus, the concentrated form of the skin care product may be packaged in a smaller container, thereby taking less space during processing, shipping, storage, and on a commercial shelf. Further, given the use of smaller packaging, there is less consumer waste and the packaging can be more environmentally friendly due to its smaller size. In addition, the present invention provides a concentrated emulsion that is stable during processing, shipping, storage, and on a commercial shelf as well after purchase by the consumer.

A concentrated composition according to the present invention may include, by way of example: a) from about 30% to about 60% by weight of water; b) at least two emulsifying agents: a first emulsifying agent and a second emulsifying agent, where the weight percent of each of the at least two emulsifying agents is from about 0.05% to about 7%, and c) from 0.1% to about 5% by weight of a thickener; where the concentrated composition is an emulsion, and where the emulsion is stable at room temperature.

### Components

The skin care product includes a combination of components to form a concentrated composition in the form of an emulsion. The concentrated product includes a first phase and second phase, which together form the concentrated emulsion. The first phase of the concentrated emulsion is in the form of a liquid, and includes water and/or other aqueous material. The first phase may also include other components such as moisturizing agents, humectants, and pH adjusters. The second phase of the concentrated emulsion (which may be considered an oily phase) includes a combination of one or more of the following: thickeners, emulsifying agents, esters, butters, oils, preservatives, surfactants, fragrances, and colors or coloring agents. The second phase may include one or more components in the form of a liquid, solid or semi-solid state. Of course, in some aspects, the first phase of the concentrated emulsion may include one or more components listed in the second phase, and the second phase may include one or more components listed in the first phase.

The concentrated form of the present skin care product includes water in an amount of from about 30% to about 60% by weight of the skin care product, or from about 35% to about 55% by weight of the skin care product, or from about 40% to about 50% by weight of the skin care product. The concentrated form of the skin care product may include an amount of water within the aforementioned ranges, including, by way of example, 37.08% or 56.8%.

It is preferred that the concentrated composition includes a thickener or thickening agent, such as carbomer, cellulose, Xanthan Gum, Acrylates/C10-30 Alkyl Acrylate Crosspolymer, Caraghenan, sodium polyacrylate, Sclerotium Gum. For example, one carbomer that may be useful in the present invention is a crosslinked polyacrylic acid, including that sold as Carbopol Ultrez 10 Polymer by Lubrizol. The thickener is present in an amount of about 0.1% to about 5% by weight of the concentrated composition, or from about 0.5% to about 3.5% by weight of the concentrated composition, or about 1.5% to about 2.5% by weight of the concentrated composition. Having a desired amount of thickener, including a polyacrylic acid thickener, in an amount that is at least 0.1% by weight of the concentrated composition is useful in maintaining the viscosity and thickness of the composition, however, when the thickener level is too great, the composition may be too viscous to be diluted by the user.

The concentrated composition may have a thickener that includes a crosslinked polyacrylic acid, or alternatively that the thickener is a crosslinked polyacrylic acid.

The concentrated composition may include one or more emulsifying agents, such as Distearyldimonium Chloride; Potassium Cetyl Phosphate; Hydrogenated Palm Glycerides; Potassium Cetyl Phosphate; Glyceryl Stearate (and) PEG-100 Stearate; Cetearyl Alcohol (and) Ceteareth-20; Cetearyl Olivate (and) Sorbitan Olivate; Glyceryl Stearate; Steareth-21; Steareth-20; Cetearyl Alcohol (and) Cetearyl Glucoside; Arachidyl Alcohol (and) Behenyl Alcohol (and) Arachidyl Glucoside;Polyglyceryl-3 Diisostearate; Polyglyceryl-2 Dipolyhydroxystearate; Steareth-2; PEG-20 Stearate; Cetearyl Isononanoate (and) Ceteareth-20 (and) Cetearyl Alcohol (and) Glyceryl Stearate (and) Glycerin (and) Ceteareth-12 (and) Cetyl Palmitate; Diisostearoyl Polyglyceryl-3 Dimer Dilinoleate; Glyceryl Stearate (and) PEG-30 Stearate; PEG-100 Stearate; Choleth-24, Ceteth-24; Steareth-10; Glyceryl Laurate; PPG-26 Buteth-26 (and) PEG-40 Hydrogenated Castor Oil; Glycerol Monostearate; PPG-2 Hydroxyethyl Cocamide; Sorbitan Trioleate; Ethylhexyl Hydroxystearate Benzoate; PEG-40 Hydrogenated Castor Oil; Sucrose Cocoate (and) ethanol; Ceteth-20; Sorbitan Stearate; Polyglyceryl-4 Isostearate; Polysorbate 65; Glycol Cetearate; Polyglyceryl-3 Polyricinoleate; Laneth-16; PEG-8 Beeswax; Oleth-30 (and) Choleth-15 (and) Ceteth-15; PEG-4 Rapeseedamide; Ceteareth-25; PPG-4-Ceteth-20; Potassium lauryl Phosphate; Lauryl Glucoside (and) Polyglyceryl-2 Dipolyhydroxystearate (and) Glycerin; Polyhydroxystearic Acid; Cetearyl Alcohol (and) Polysorbate 60; C14-22 Alcohols (and) C12-20 Alkyl Glucoside; Cetearyl Alcohol (and) Dicetyl Phosphate (and) Ceteth-10 Phosphate; Sorbitan Sesquioleate; Polyglyceryl-4 Laurate/Sebacate (and) Polyglyceryl-6 Caprylate/Caprate; PPG-5-Ceteth-20; PEG-30 Dipolyhydroxystearate; Cetyl Alcohol (and) Glyceryl Stearate (and) PEG-75 Stearate (and) Ceteth-20 (and) Steareth-20; Steareth-100 (and) Steareth-2 (and) Mannan (and) Xanthan Gum; Ceteareth-6 (and) Stearyl Alcohol; Sorbitan Isostearate; Methyl Glucose Isostearate; Sorbitan Stearate (and) Sucrose Cocoate; Polyglyceryl-2 Isostearate; Isoceteth-20; Laureth-4; Zinc stearate; Stearyl Alcohol (and) Ceteareth-20; Disodium Cetearyl Sulfosuccinate; Polyglyceryl-3 Caprylate; Stearyl Alcohol (and) Ceteareth 20; Sucrose Cocoate, and combinations thereof. Emulsifiers such as hydrogenated palm glycerides and potassium cetyl phosphate are particularly useful emulsifiers, whether used alone or used in combination with each other.

It may be desired that the concentrated composition includes a first emulsifying agent and a second emulsifying agent. For example, the concentrated composition may include a first emulsifying agent that is potassium cetyl phosphate and a second emulsifying agent that is hydrogenated palm glycerides. The emulsifying agent or agents may be used in combined amounts of from about 0.05% to about 9% by weight of the concentrated composition. When two emulsifying agents are used, they may be included separately in the composition and/or they may be first combined together. Further, the emulsifying agents may be combined together prior to use in forming the concentrated skin care composition, and separately one or both of the emulsifying agents may be additionally included in the concentrated skin care composition. For example, a concentrated skin care composition may include the first emulsifying agent and second emulsifying agent that have been combined together as a pre-mixed composition, and one of or both of the first and second emulsifying agents may also be included in the concentrated skin care composition independently.

The concentrated composition may have a first emulsifying agent including potassium cetyl phosphate. In addition, or alternatively, the concentrated composition may have a second emulsifying agent including hydrogenated palm glycerides.

Each emulsifying agent may independently be present in an amount of about 0.05% to about 7% by weight of the concentrated composition, or about 0.05% to about 4% by weight of the concentrated composition, or about 0.5% to about 2% by weight of the concentrated composition, or about 0.5% to about 1% by weight of the concentrated composition. In particular, if potassium cetyl phosphate is used as one of the emulsifying agents, it may be present in amounts of from about 0.5% to about 7% by weight of the concentrated composition, or about 1.5% to about 4% by weight of the concentrated composition, or about 2% to about 3.5% by weight of the concentrated composition. In particular, if hydrogenated palm glycerides is used as one of the emulsifying agents, it may be present in amounts of from about 0.05% to less than about 4% by weight of the concentrated composition, or from about 0.05% to about 3.5% by weight of the concentrated composition, or from about 0.05% to about 3% by weight of the concentrated composition, or from about 0.5% to about 1.5% by weight of the concentrated composition, or from about 0.5% to about 1% by weight of the concentrated composition.

The concentrated composition may have a first emulsifying agent present from about 0.5% to about 7%, or from about 1.5% to about 4%, or from about 2% to about 3.5% by weight of the concentrated composition.

The concentrated composition may have a second emulsifying agent present from about 0.05% to about 3%, or from about 0.5% to about 1.5%, or from about 0.5% to about 1% by weight of the concentrated composition.

When the concentrated composition includes a first emulsifying agent and a second emulsifying agent, the first and second emulsifying agents may be present in weight ratio of about 1:1 to 27:1, or more specifically around 3:1. For example, when the first emulsifying agent is potassium cetyl phosphate and the second emulsifying agent is hydrogenated palm glycerides, the first and second emulsifying agents may be present in ratio by weight of about 1:1 to 27:1, or about 3:1. If used together, it may be desired that the weight amount of potassium cetyl phosphate be greater than the amount of hydrogenated palm glycerides. In particular, the use of emulsifiers such as potassium cetyl phosphate and hydrogenated palm glycerides aids in the stability of the concentrated product and resulting diluted product.

The concentrated composition may have a weight amount of the first emulsifying agents that is superior or equal to the weight ration of the second emulsifying agents.

The concentrated composition may have a first emulsifying agent and a second emulsifying agent, where the first emulsifying agent is present from about 0.5% to about 7%, or from about 1.5% to about 4%, or from about 2% to about 3.5% by weight of the concentrated composition; and where the second emulsifying agent is present from about 0.05% to about 3%, or from about 0.5% to about 1.5%, or from about 0.5% to about 1% by weight of the concentrated composition.

Preferably, the concentrated composition may have a first emulsifying agent and a second emulsifying agent where the first emulsifying agent is present from about 2% to about 3.5% by weight of the concentrated composition; and where the second emulsifying agent is present from about 0.5% to about 1% by weight of the concentrated composition.

When the concentrated composition includes a thickener and an emulsifying agent or emulsifying agents, the thickener and emulsifying agent(s) may be present in a weight ratio of each other. For example, when potassium cetyl phosphate and carbomer are used, they may be present in a weight ratio of 0.6:1 to 3:1, or about 1:1. When a thickener, such as a polyacrylic acid thickener, and hydrogenated palm glycerides are used, they may be present in a weight ratio of from 1.5:1 to 10:1, or about 3:1 to 3.5:1. Thus, it is desired that a thickener, such as a polyacrylic acid thickener be present in a greater weight amount than hydrogenated palm glycerides.

The concentrated composition may have a weight amount of the thickener that is superior or equal to the weight ratio of the second emulsifying agents.

The concentrated composition may include pH adjusting agents, such as sodium hydroxide, Triethanolamine, Citric Acid, Sodium Citrate, lactic acid, and combinations thereof. When used, a pH adjusting agent may be present in an amount from greater than zero to about 1.7% by weight of the concentrated composition, or from 0.01% to about 1.7% by weight of the concentrated composition.

The concentrated composition may include preservatives, such as phenoxyethanol, Tropolone, Chlorphenesin, Parabens, Potassium Sorbate, Sodium Benzoate, DMDM Hydantoin Chlorhexidine, Benzyl alcohol, sorbic acid, p-anisic acid, and combinations thereof. When used, a preservative may be present in an amount from greater than zero to about 2% by weight of the concentrated composition, or from 0.01% to about 2% by weight of the concentrated composition.

The concentrated skin care product may include humectants or moisturizing agents, such as glycerin, Propylene Glycol, Butylene Glycol, Pentylene Glycol, Sorbitol, Sucralose, Sodium Saccharin, and combinations thereof. When used, a humectant may be present in an amount from greater than zero to about 30% by weight of the concentrated composition, or from about 10% to about 30% by weight of the concentrated composition.

Preferably, the concentrated composition may include glycerin as a humectant.

The concentrated composition may include glycerin as a humectant, where the glycerin may be present in an amount from about 10% to about 30% by weight of the concentrated composition.

The concentrated composition may include one or more skin conditioning agents, including conditioners, emollients, and oils. Skin conditioning agents may include such agents as butters such as shea butter, panthenol, silicones, oils such as mineral oil, hydrogenated castor oil, and vegetable oil, petrolatum, esters such as isopropyl palmitate and isopropyl myristate, caprylyl glycol, liquid paraffin, glycerin, and combinations thereof.

When used, an individual skin conditioning agent may be present in an amount from greater than zero to about 35% by weight of the concentrated composition, or from about 0.1% to about 20% by weight of the concentrated composition, or from about 1% to about 5% by weight of the concentrated composition. Combinations of skin conditioning agents may be used, which may have a combined weight of from about 0.1% to about 50% by weight of the concentrated composition, or from about 0.5% to about 35% by weight of the concentrated composition, or from about 1% to about 15% by weight of the concentrated composition.

As noted, combinations of skin conditioning agents may be used in the concentrated composition, such as the combination of glycerin, liquid paraffin, hydrogenated castor oil, and caprylyl glycol.

Some concentrated compositions may include one or more additional active ingredients, such as UV filters, film formers, acids such as Salicylic acid, AHA, BHA, PHA, Hyaluronic acid, or retinol. It may be desired that the concentrated composition be free of one or more of the aforementioned additional active ingredients, or that any desired additional active ingredients be added separately by the consumer, either prior to or after dilution, as described below.

### Kit

In some aspects, the invention may also relate to a skin care product kit that may include a container including at least a first composition and a second composition, where a) the first composition includes a concentrated composition according to the present invention, and b) the second composition includes at least one of a texture ingredient and/or a skin active ingredient, where the first composition and the second composition can be mixed together with water, either before or after being combined together, to obtain a skin care product capable of being applied to the skin of the user.

The concentrated form of the skin care product may be provided to a user within a kit to allow the user to prepare the formulation and ultimately use the product. The kit may include one container or package, or it may include multiple containers or packages, each including contents therein. The contents may include one or more containers for use in dispensing, mixing, and/or using the product. A first container may be included, which contains an amount of the skin care product in the concentrated form. The amount of the skin care product may be suitable for a single use, or for multiple uses. The container in which the concentrated form of the skin care product may be resealable or may be intended to be opened a single time. The container may be ajar, bottle, box, pouch, bag, or any other desired container that maintains the concentrated form of the skin care product therein. The user may add water directly to the first container and mix the concentrated form of the skin care product contained therein with water to form the diluted product.

If the concentrated form of the skin care product is intended to be used multiple times, there may be provided a means for measuring the concentrated composition, such as a spoon, bottle, dropper, pump, or other means to allow a user to remove a desired amount of the concentrated composition from the first container.

The contents of the kit may further include a second container, which may be empty, in which the user may dilute the desired portion of the skin care product in its concentrated form. Thus, the user may take all or a portion of the concentrated composition from the first container and place it into the second container. The second container may have indicia identifying for the user how much product should be placed therein. Alternatively, there may be a measuring tool provided to allow the user to place a desired amount of the skin care product in its concentrated form into the second container. The user may add a desired amount of water to the second container, either prior to adding the concentrated form of the skin care product or after adding the concentrated form of the skin care product. The user may then mix the water and concentrated form of the skin care product together as described herein.

The contents of the kit may further include a third container, which includes one or more texture ingredients as shea butter, Sodium Polyacrylate, Waxes, oils, Carbomer, and the like. The texture ingredients may be present in an amount to be used a single time or may be present in an amount to be used multiple times. The user may remove a desired amount of the texture ingredient or ingredients from the third container and add the desired amount of texture ingredients to be mixed with the water and the concentrated form of the skin care product. The texture ingredients may be added in the first container or in the second container, and the texture ingredients may be added prior to addition of water or after addition of water.

The contents of the kit may further include a fourth container, which includes one or more additional skin active ingredients, such as skin active components that provide a medical, therapeutic, or other benefit to the skin to which the product is applied. The additional active ingredients may be present in an amount to be used a single time or may be present in an amount to be used multiple times. The user may remove a desired amount of the additional active ingredient or ingredients from the fourth container and add the desired amount of additional active ingredients to be mixed with the water and the concentrated form of the skin care product. The additional active ingredients may be added in the first container or in the second container, and the additional active ingredients may be added prior to addition of water or after addition of water.

The contents of the kit may further include one or more instructions for use, including identifying the amount of concentrated composition, water, active ingredient, and/or texture ingredient to be mixed together. The kit may include instructions identifying the method of mixing, which may be any method described herein.

The contents of the kit may include a means for stirring the components, such as a spoon, a stick, a rod, or other tool to allow for hand stirring without the need for a motorized or electromechanical mixing apparatus.

The skin care product kit may contain a plurality of compositions that may be mixed with water, at room temperature, without the need for a motorized or electromechanical mixing apparatus.

### Method of diluting

As indicated above, the first form of the skin care product is provided to a user as a concentrated emulsion, and prior to applying the product to the skin or hair, the user dilutes the concentrated form of the skin care product with water or other aqueous material, mixing the combination sufficiently to result in the second form of the skin care product. The second form of the skin care product may then be applied to the skin or hair of the user to provide the desired benefit. In some aspects, as described above, there may be an active composition that is separately added, and the active composition may be added to the concentrated form of the first product, the water, or the combination of the concentrated form of the first product and water, either before or after mixing. A step of mixing should occur after the active composition is added, even if the concentrated form of the skin care product and water have been mixed to form the second form of the skin care product.

The process of diluting the concentrated form of the skin care product to result in the second form of the skin care product includes the steps of adding an amount of an aqueous material, such as water, to the concentrated form of the skin care product, and mixing the concentrated form of the skin care product. As will be described below, the dilution should include the addition of a desired amount of aqueous material, which is mixed by the user. The mixing of the concentrated form of the skin care product and aqueous material is capable of being effectively performed without the need for complicated machinery or electromechanical apparatuses. Further, the mixing may be capable of being performed effectively at room temperature, avoiding the need to heat and/or cool the components either before or after mixing.

The aqueous product may include water. The water may be a temperature of from about 15°C to 35°C, or about 15°C to 30°C, about 15°C to 25 °C, or from about 18°C to 23 °C, or about 20°C to 22 °C. Temperatures are expressed in Celsius degrees. Water may be added in any desired means, such as through the use of a measuring apparatus, such as a beaker, spoon, dropper, vial, or any other apparatus that identifies the amount of water being added. The concentrated form of the skin care product may be provided in a container in which the water can be directly added, or the concentrated form of the skin care product may be dispensed from its initial container into a second container, into which the water may be added.

The invention also relates to a diluted skin care composition that may include the combination of: a) a concentrated composition according to the present invention, and b) an aqueous component, where the aqueous component may be present in a weight ratio of from about 1 to 20 times the weight of the concentrated composition, and where the aqueous component may include at least 95% water.

Preferably the diluted skin care composition may include an aqueous component present in a weight ratio of from about 2 to 17 times, preferably 3 to 12 times the weight of the concentrated composition. More preferably the weight ratio of said aqueous component to the concentrate composition may range from 1 to 16 times, or 2 to 16 times, or 1 to 11 times, or 2 to 11 times, or 3 to 16 times, or 3 to 11 times; even more preferably from 1 to 5 times, or from 2 to 5 times, or from 2 to 4 times.

The diluted composition may have a weight ratio of said aqueous component to the concentrate composition ranging from 1 to 2 times, or from 2 to 3 times, or from 4 to 5 times, or from 10 to 11 times, or from 15 to 17 times.

Preferably the diluted skin care composition may include an aqueous component that may include at least 99%, preferably 99.5% of water.

The diluted composition may include the aqueous component which is present in an amount of from 4 to 45 times the amount of water that is present in the concentrated composition.

The present invention allows for a significant amount of water to be added to the concentrated form of the skin care product, and yet still provide a diluted product that has a sufficiently high viscosity without breaking the emulsion and/or reducing stability of the diluted product. The final diluted product, which includes the concentrated form of the product and water (and optionally texture ingredients and/or skin active ingredients, as discussed above), is stable, has a sufficient viscosity, and is suitable for use on the skin of the user. It is desired that the amount of water added by a user to dilute the concentrated form of the skin care product may be about 4-5 times the amount of water in the concentrated form of the skin care product, or may be about 7-10 times the amount of water in the concentrated form of the skin care product, or may be about 10-15 times the amount of water in the concentrated form of the skin care product, or may be about 25-30 times the amount of water in the concentrated form of the skin care product, or may be about 40-45 times the amount of water in the concentrated form of the skin care product.

Water may be added to the concentrated form of the product in a weight amount that is about 2 times the weight amount of the concentrated skin care composition. For example, if the concentrated skin care composition is 25 grams, water may be added in an amount of about 50 grams. As used herein, this is termed a "75% dilution".

Water may be added in a weight amount that is about 3 times the weight amount of the concentrated skin care composition. For example, if the concentrated skin care composition is 25 grams, water may be added in an amount of about 75 grams. As used herein, this is termed a "100% dilution".

Water may be added to the concentrated form of the product in a weight amount that is about 5 times the weight amount of the concentrated skin care composition. For example, if the concentrated skin care composition is 25 grams, water may be added in an amount of about 125 grams. As used herein, this is termed a "150% dilution".

Water may be added to the concentrated form of the product in a weight amount that is about 11 times the weight amount of the concentrated skin care composition. For example, if the concentrated skin care composition is 25 grams, water may be added in an amount of about 275 grams. As used herein, this is termed a "300% dilution".

Water may be added to the concentrated form of the product in a weight amount that is about 17 times the weight amount of the concentrated skin care composition. For example, if the concentrated skin care composition is 25 grams, water may be added in an amount of about 425 grams. As used herein, this is termed a "450% dilution".

The user may add a significant amount of water to dilute the concentrated skin care composition to produce the diluted skin care product. Even with the addition of water in an amount of about 40 to about 45 times the amount of water contained in the concentrated composition, the resulting viscosity of the diluted skin care product is above 300 cP at room temperature. It would be expected that the addition of such a high amount of water would result in a viscosity that is too thin to be used for certain purposes, such as creams, gels or balms (e.g., below 300 cP, or below 500 cP, or below 750 cP), but the present invention allows for a resulting diluted product to remain sufficiently viscous.

As noted above, concentrate in the form of an emulsion would be expected to be difficult to dilute and still provide a useful product because of the lack of stability between the phases. In the present invention, the viscosity of the resulting diluted skin care product is sufficiently high to be used as a thick product, such as a balm, gel, lotion, or cream. The resulting viscosity may be from 300 cP to 4,500 cP at room temperature, or may be from about 500 cP to about 3,500 cP at room temperature, or may be from about 750 cP to about 2,000 cP at room temperature, depending upon the amount of water added to dilute the concentrated form of the skin care product. Adding a module with a viscosing agent can lead to even higher viscosity levels, if desired.

In addition, as noted above, the step of mixing the concentrated form of the skin care product and water to achieve the second form of the skin care product is simple and can be performed by the user without the need for complicated machinery or electromechanical devices. After adding the desired amount of aqueous material to the concentrated form of the skin care product, the concentrated product and the water are mixed together by the user to form the second form of the skin care product. As noted above, concentrated emulsions are typically difficult to mix and maintain stability, typically requiring motorized equipment or other methods that give a high mixing speed. Typical emulsions require motorized or sophisticated equipment such as deflocculators and mixers with blades rotating at high RPM's to achieve thorough mixing and result in a stable end product, and/or require mixing at specific temperatures, typically elevated above room temperature. With the present invention, however, mixing is easier and only requires a lower speed of mixing, which may be achieved through hand mixing with a simple device such as a spoon, stick, finger, or stirring rod, and allows for mixing to be achieved at room temperature. This mixing process allows for mixing without the need to modify temperature of any ingredients.

Mixing of the concentrated form of the skin care product and water may be achieved by the user's hand, using a non-motorized element such as a spoon, stick, finger, or stirring rod, and may be completed to provide a sufficiently mixed and diluted skin care product within less than 5 minutes of mixing, or less than 3 minutes of mixing, or less than 1 minute of mixing.

After mixing is complete, the diluted, second form of the skin care product is formed, where the second form may be applied to the skin or hair of the user. The second form has a viscosity that is sufficiently high for use as a thicker skin care product, such as a balm, gel, lotion, or cream, as described above.

The invention also relates to a method of forming a diluted skin care product that may include the steps of combining together: a) a concentrated composition that may include: i) from about 30% to about 60% by weight of water, ii) at least two emulsifying agents: a first emulsifying agent and a second emulsifying agent, where the weight percent of each of the at least two emulsifying agents may independently be from about 0.05% to about 7%, and iii) from 0.1% to about 5% by weight of a thickener; where the concentrated composition may be an emulsion, the emulsion being stable at room temperature; and b) an aqueous component, where the combined concentrated composition and aqueous component may be mixed with each other to form a diluted skin care product.

Preferably, the method combining step may be performed at a temperature from 15°C to 35°C, preferably 15°C to 30°C, preferably 15°C to 25°C. The method may include the aqueous component to be provided in a weight ratio of from 1 to 20 times the weight of the concentrated composition. Preferably the aqueous component may be provided in a weight ratio of from 2 to 17 times, or from 3 to 12 times the weight of the concentrated composition.

### Use of Diluted Skin Care Product

In another aspect the invention may relate to the use of a concentrated composition according to the description above, for the preparation of a diluted skin care product.

The second form of the skin care product is the form of the skin care product that is applied to the hair or skin of the user to provide a benefit to that user, also known as the skin-applied form, or the diluted form of the skin care product. The benefit to be provided to the skin or hair of the user may include one or more of moisturizing, barrier strengthening, conditioning of the hair, deposition of benefit or therapeutic agents, or combinations of these benefits. These agents may be classified as "skin benefit agents", as discussed above.

If the second form of the skin care product is intended to or does moisturize the skin or provide barrier protection to the skin, the skin-applied form of the skin care product may include one or more moisturizing agents.

The invention may relate to the use of a concentrated composition for the preparation of a diluted skin care moisturizer.

The concentrated composition according to the description above may be implemented for the preparation of a diluted skin care product used as a skin moisturizer.

The diluted skin care product may be used as a cosmetic product.

Furthermore, the invention may relate to the use of a diluted skin care product prepared according to the method described above, for the moisturizing of skin.

If the second form of the skin care product is intended to or does condition the hair of the user, the skin-applied form of the skin care product may include one or more conditioning agents.

If the second form of the skin care product is intended to or does deposit or otherwise provide one or more benefit or therapeutic agents to the skin or hair of the user, the skin-applied form of the skin care product may include one or more therapeutic agents.

As noted above, the concentrated form of the skin care product may include one or more skin benefit agents when provided to the user, or the concentrated form of the skin care product may be free of one or more skin benefits agents, where the skin benefit agent(s) are intended to be added separately by the user. In aspects where the concentrated form of the skin care product includes one or more benefit agents, after adding the desired amount of water to the concentrated form of the skin care product and mixing the water and the concentrated form of the skin care product so as to form the diluted form of the skin care product, the user may apply a desired amount of the diluted form of the skin care product to the skin or hair of the user.

In aspects where the concentrated form of the skin care product does not include one or more skin benefit agents when provided to the user, it may be desired that one or more skin benefit agent or agents be added by the user, either before dilution with water or after dilution with water. In this embodiment, the combination of the concentrated composition, skin benefit agent(s) and water forms the final skin-applied form of the skin care product, and may be used by the user. Note that in this embodiment, the concentrated form of the skin care product may include one or more skin benefit agents at the time it is provided to the user, but the user may add an additional skin benefit agent or agents, as described herein.

### Examples

### Example 1 - concentrated skin care compositions

Several exemplary concentrated compositions were formed and tested for stability, viscosity, stickiness, and overall usability as concentrated emulsions. The amounts of ingredients in Tables 1-4 are shown in percentage by weight of the concentrated product.

**Table 1: Inventive Examples E1-E6**

| | E1 | E2 | E3 | E4 | E5 |
|---|---|---|---|---|---|
| Water | 55.25% | 55.25% | 40.61% | 49.23% | 56.80% |
| Carbomer | 1.38% | 1.38% | 2.68% | 3.59% | 4.14% |
| Glycerin | 13.81% | 13.81% | 19.16% | 25.64% | 29.59% |
| Caprylyl Glycol | 0.83% | 0.83% | 1.15% | 1.54% | 1.78% |
| p-Anisic Acid | 0.28% | 0.28% | 0.38% | 0.51% | 0.59% |
| Sodium Hydroxide (32%); Water (68%) | 1.66% | 1.66% | 0.77% | 3.08% | - |
| Hydrogenated Palm Glycerides (50%); Potassium Cetyl Phosphate (50%) | 0.28% | 0.28% | 0.77% | 4.62% | 5.33% |
| Paraffinum Liquidum | 22.10% | 22.10% | 30.65% | 10.26% | - |
| Phenoxyethanol | 0.83% | 0.83% | 1.15% | 1.54% | 1.78% |
| Potassium Cetyl Phosphate | 3.59% | 3.59% | 2.68% | - | - |
| Hydrogenated Castor Oil | - | - | - | - | - |
| | | | | | |
| Viscosity (cP, room temp) | 4,000 | 4,650 | 8,810 | 10,000 | 9,000 |

**Table 2: Inventive Examples E7-E11**

| | E6 | E7 | E8 | E9 | E10 | E11 |
|---|---|---|---|---|---|---|
| Water | 50.79% | 37.08% | 46.97% | 46.53% | 47.86% | 43.88 |
| Carbomer | 3.70% | 2.60% | 2.35% | 2.33% | 0.50% | 2.20 |
| Glycerin | 26.46% | 18.59% | 16.78% | 16.63% | 17.10% | 15.67 |
| Caprylyl Glycol | 1.59% | 1.12% | 1.01% | 1.00% | 1.03% | 0.94 |
| p-Anisic Acid | 0.53% | 0.37% | - | - | - | |
| Sodium Hydroxide (32%); Water (68%) | - | 2.32% | 2.01% | 1.99% | 2.05% | 1.88 |
| Hyd rogenated Palm Glycerides (50%); Potassium Cetyl Phosphate (50%) | 4.76% | 3.35% | 1.51% | 1.50% | 1.54% | 8.00 |
| Paraffinum Liquidum | 10.58% | 29.74% | 26.85% | 26.61% | 27.36% | 25.08 |
| Phenoxyethanol | 1.59% | 1.12% | 1.01% | 1.91% | 1.03% | 0.94 |
| Potassium Cetyl Phosphate | | - | 1.51% | 1.50% | 1.54% | 1.41 |
| Hyd rogenated Castor Oil | | 3.72% | - | - | - | |
| | | | | | | |
| Viscosity (cP, room temp) | 5,420 | 13,000 | 10,000 | 6,500 | 646 | - |

**Table 3: Comparative Examples C1-C3**

| | C1 | C2 | C3 |
|---|---|---|---|
| Water | 25 | 48.1 | 45.21 |
| Carbomer | 3.32 | - | 6.0 |
| Glycerin | 23.73 | 17.18 | 16.15 |
| Caprylyl Glycol | 1.43 | 1.03 | 0.97 |
| p-Anisic Acid | - | - | - |
| Sodium Hydroxide (32%); Water (68%) | 2.84 | 2.06 | 1.93 |
| Hydrogenated Palm Glycerides (50%); Potassium Cetyl Phosphate (50%) | 2.14 | 1.55 | 1.45 |
| Paraffinum Liquidum | 37.97 | 27.50 | 25.85 |
| Phenoxyethanol | 1.43 | 1.03 | 0.97 |
| Potassium Cetyl Phosphate | 2.14 | 1.55 | 1.45 |
| Hydrogenated Castor Oil | - | - | - |

**Table 4: Comparative Examples C4-C5**

| | C4 | C5 |
|---|---|---|
| Water | 48.43 | 43.25 |
| Carbomer | 2.42 | 2.16 |
| Glycerin | 17.30 | 15.45 |
| Caprylyl Glycol | 1.04 | 0.93 |
| p-Anisic Acid | | |
| Sodium Hydroxide (32%); Water (68%) | 2.07 | 1.85 |
| Hydrogenated Palm Glycerides (50%); Potassium Cetyl Phosphate (50%) | - | 1.39 |
| Paraffinum Liquidum | 27.69 | 24.73 |
| Phenoxyethanol | 1.04 | 0.93 |
| Potassium Cetyl Phosphate | - | 9.30 |
| Hydrogenated Castor Oil | - | - |

Examples E1-E10 were deemed to be satisfactory as concentrated emulsions, demonstrating sufficient stability without phase separation, and each having viscosity levels identified in the table. Example E11 was stable, but was considered to be too viscous to be used in a pumpable formulation, which may be due to the higher amount of hydrogenated palm glycerides (4%). It is noted that a higher viscosity may be desired in some instances, and since E11 was stable, it may be satisfactory in such instances.

Comparative Examples C1-C5 were deemed unsatisfactory as a concentrated emulsion for various reasons, including, for example, lack of stability as a concentrated product or lack of ability to be diluted properly. Comparative Examples C1 and C3, for example, did not include a sufficient level of water in the concentrated form to hydrate the amount of thickener present. Comparative Example C2, for example, lacked a stable emulsion, and demonstrated phase separation upon completion of mixing. It is believed that this lack of stability was due to the lack of thickener in the composition. Comparative Example C5 was stable, but was too viscous. It is believed that the Comparative Example C5 included too high a level of potassium cetyl phosphate (10% by weight). Comparative Example C4 demonstrated an emulsion that was not stable, demonstrating phase separation upon completion of mixing. It is believed that the lack of hydrogenated palm glycerides and/or potassium cetyl phosphate was a factor in this lack of stability.

### Example 2 - Diluted Concentrated Bases

Various concentrated compositions were formed and then diluted with various amounts of water. For each of Examples DC1-DC5, the concentrated form of the product was deemed stable with a sufficient viscosity level. The amounts reflected in the table are the percentage of components in the resulting diluted product after water has been added. For Example DC1, the full amount of components in the resulting diluted product equals 75%, for DC2, the full amount of components in the resulting diluted product equals 100%, for DC3, the full amount of components equals 150%, for DC4, the full amount of components in the resulting diluted product equals 300%, and for DC5, the full amount of components in the resulting diluted product equals 450%.

**Table 5: Inventive Examples DC1-DC5**

| | DC1 | DC2 | DC3 | DC4 | DC5 |
|---|---|---|---|---|---|
| Water | 9.575 | 9.575 | 9.575 | 9.575 | 9.575 |
| Carbomer | 0.7 | 0.7 | 0.7 | 0.7 | 0.7 |
| Glycerin | 5 | 5 | 5 | 5 | 5 |
| Caprylyl Glycol | 0.3 | 0.3 | 0.3 | 0.3 | 0.3 |
| p-Anisic Acid | 0.1 | 0.1 | 0.1 | 0.1 | 0.1 |
| Sodium Hydroxide (32%); Water (68%) | 0.625 | 0.625 | 0.625 | 0.625 | 0.625 |
| Hyd rogenated Palm Glycerides (50%); Potassium Cetyl Phosphate (50%) | 0.9 | 0.9 | 0.9 | 0.9 | 0.9 |
| Paraffinum Liquidum | 8 | 8 | 8 | 8 | 8 |
| Phenoxyethanol | 0.3 | 0.3 | 0.3 | 0.3 | 0.3 |
| | | | | | |
| | | | | | |
| Sub total | 25.5 | 25.5 | 25.5 | 25.5 | 25.5 |
| Concentrated Viscosity Range (cP) | 1600-3000 | 1600-3000 | 1600-3000 | 1600-3000 | 1600-3000 |
| | | | | | |
| | | | | | |
| Dilution - water | 49.5 | 74.5 | 125 | 275 | 424.5 |
| | | | | | |
| Diluted pH | 5.66 | 5.7 | 5.84 | 6.06 | 6.3 |
| Diluted Viscosity (cp) | 4440 | 3500 | 1990 | 727 | 315 |

The concentrated viscosity of the DC1-DC5 examples differed between batches of the example, with the viscosity being between approximately 1600-3000 for each example. The resulting viscosity levels after dilution are reflected in the table above (in cP at room temperature). As can be seen, the resulting viscosity level for DC1 was 4,440 cP, the resulting viscosity level for DC2 was 3,500 cP, the resulting viscosity level for DC3 was 1,990 cP, the resulting viscosity level for DC4 was 727 cP, the resulting viscosity level for DC5 was 315 cP. Even with the addition of 44.3 times the amount of water (the amount of water added to dilute compared the amount of water in the concentrated base), the resulting viscosity was above 300 cP. The resulting diluted product was sufficiently stable and had a sufficient viscosity level to be used as described herein.

## Claims

1. A concentrated composition including:
a) From about 30% to about 60% by weight of water;
b) At least two emulsifying agents: a first emulsifying agent and a second emulsifying agent, wherein the weight percent of each of said at least two emulsifying agents is from about 0.05% to about 7%, and
c) From 0.1% to about 5% by weight of a thickener;
Wherein said concentrated composition is an emulsion.

2. The concentrated composition according to claim 1, wherein said first emulsifying agent comprises potassium cetyl phosphate.

3. The concentrated composition according to claim 1 or claim 2, wherein said second emulsifying agent comprises hydrogenated palm glycerides.

4. The concentrated composition according to any of claims 1 to 3, wherein said first emulsifying agent is present from about 0.5% to about 7% by weight of the concentrated composition.

5. The concentrated composition according to any of claims 1 to 4, wherein said second emulsifying agent is present from about 0.05% to about 3% by weight of the concentrated composition.

6. The concentrated composition according to any of claims 1 to 5, wherein said thickener comprises crosslinked polyacrylic acid.

7. The concentrated composition according to any of claims 1 to 6, wherein the concentrated composition comprises a humectant.

8. The concentrated composition according to claim 7, wherein said humectant is present in an amount from greater than 0% to about 30% by weight of the concentrated composition.

9. A skin care product kit comprising a container including at least a first composition and a second composition, wherein
a) The first composition comprises a concentrated composition according to any of claims 1 to 8, and
b) The second composition comprises at least one of a texture ingredient or a skin active ingredient,
wherein said first composition and said second composition can be mixed together with water, to obtain a skin care product capable of being applied to the skin of the user.

10. A diluted skin care composition, including the combination of:
a) a concentrated composition according to any of claims 1 to 8, and
b) an aqueous component,
wherein said aqueous component is present in a weight ratio of from about 1 to 20 times the weight of the concentrated composition, and
wherein said aqueous component comprises at least 95% water.

11. A diluted composition according to claim 10, wherein the aqueous component is present in an amount of from 4 to 45 times the amount of water that is present in the concentrated composition.

12. A method of forming a diluted skin care product, comprising the steps of combining together:
a) a concentrated composition comprising:
i) From about 30% to about 60% by weight of water,
ii) At least two emulsifying agents: a first emulsifying agent and a second emulsifying agent, wherein the weight percent of each of said at least two emulsifying agents is from about 0.05% to about 7%, and
iii) From 0.1% to about 5% by weight of a thickener;
Wherein said concentrated composition is an emulsion; and
b) an aqueous component,
wherein the combined concentrated composition and aqueous component are mixed with each other to form a diluted skin care product.

13. The method according to claim 12, wherein the aqueous component is provided in a weight ratio of from 1 to 20 times the weight of the concentrated composition.

14. Use of a concentrated composition according to any of claims 1 to 8, for the preparation of a diluted skin care product.

15. Use of a diluted skin care product prepared according to the method of claim 12, for the moisturizing of skin.
